# EUROPÄISCHE PATENTSCHRIFT

(11) **EP 2 173 330 B1**
(45) Veröffentlichungstag und Bekanntmachung des Hinweises auf die Patenterteilung: **04.04.2012**
(21) Anmeldenummer: 08784299.3
(22) Anmeldetag: 04.07.2008
(51) Int. Cl.: A61K 9/70, A61L 15/44

(54) **RESERVOIRSYSTEM MIT VERSCHLOSSENER MEMBRAN**
RESERVOIR SYSTEM COMPRISING A CLOSED MEMBRANE
SYSTÈME À RÉSERVOIR MUNI D'UNE MEMBRANE FERMÉE

(30) Priorität: 04.07.2007 DE 102007030965
(43) Veröffentlichungstag der Anmeldung: 14.04.2010
(73) Patentinhaber: Acino AG, 83714 Miesbach (DE)
(72) Erfinder: HAUSNER, Heike, 83607 Holzkirchen (DE); BRAUN, Sebastian, 42929 Wermelskirchen (DE); SPILGIES, Heiko, 81373 München (DE)
(74) Vertreter: Beetz & Partner
(86) Internationale Anmeldenummer: PCT/DE2008/001112
(87) Internationale Veröffentlichungsnummer: WO 2009/003466

(56) Entgegenhaltungen:
- WO-A-00/47208
- WO-A-00/59483
- WO-A-99/32095
- WO-A-03/011291
- GB-A- 2 402 884

## Beschreibung

Gegenstand der Erfindung ist ein dermales oder transdermales therapeutisches System, das ein Reservoir mit mindestens einem Wirkstoff, eine das Wirkstoffreservoir begrenzende wirkstoffdurchlässige Membran und eine Verschlussschicht aufweist, wobei die Verschlussschicht für den mindestens einen Wirkstoff bei einer Temperatur unterhalb der Hauttemperatur impermeabel und bei einer Temperatur von Hauttemperatur oder darüber permeabel ist.

Therapeutische Systeme zur Verabreichung von Wirkstoffen mittels Absorption durch die Haut (dermale oder transdermale Verabreichung) haben in den vergangenen Jahrzehnten eine hohe Bedeutung erlangt. Mit der Zeit ist eine immer größer werdende Zahl von dermal bzw. transdermal applizierbaren Arzneimitteln bekannt geworden. Der Vorteil dieser Verabreichungsform besteht im Übergang von Wirkstoffen in die Blutgefäße unter Umgehung des gastrointestinalen Trakts. Die Passage durch das hepatische System wird also vermieden, bevor der Wirkstoff an sein Ziel gelangt.

Die genannten Systeme, wie Pflaster, kann man bezüglich ihrer Funktionsweise in zwei Hauptkategorien einteilen - sogenannte Matrixsysteme und Reservoirsysteme. Erstere enthalten den Wirkstoff oder die Wirkstoffformulierung in der Klebschicht, zur zweiten Kategorie gehören solche Systeme, welche den Wirkstoff oder die Wirkstoffformulierung in einem Reservoir enthalten, in dessen Füllmaterial der Wirkstoff üblicherweise dispergiert vorliegt. Das Füllmaterial des Reservoirsystems kann aus einem Polymer und/oder aus einer Flüssigkeit bestehen. Zur Vermeidung des Auslaufens der wirkstoffhaltigen Flüssigkeit sind dermale oder transdermale therapeutische Systeme vom Reservoirtyp für gewöhnlich von einer wirkstoffdurchlässigen Membran, die hautseitig mit einer Kleberschicht versehen ist, begrenzt. Solche Systeme sind z. B. in den Patentschriften US 4 379 454, WO 03/011 291, EP 0 366 240 sowie DE 689 29 533 beschrieben.

Trotz vorteilhafterAnwendungsmöglichkeiten haben die bekannten Reservoirsysteme auch Nachteile. Aufgrund der zumeist nur geringen Stabilität der hautseitigen Kleberschicht in Gegenwart von Flüssigkeiten ist die Lagerstabilität häufig problematisch. Eine Instabilität der Kleberschicht durch Kontakt mit der Flüssigkeit kann zum Auslaufen des Reservoirs führen und somit zu einem nicht verwendbaren System. Des Weiteren stellt die hautseitige Kleberschicht für Wirkstoffe eine zusätzliche Barriere bei der Permeation des Wirkstoffes aus dem Reservoir dar, was vor allem bei Wirkstoffen mit einer hohen benötigten Dosis ein begrenzender Faktor sein kann. Bei sehr potenten Wirkstoffen mit einem engen therapeutischen Index kann hingegen die Anreicherung des Wirkstoffes in der Kleberschicht zu einer unerwünschten zu hohen initialen Freisetzung des Wirkstoffes nach Applikation des Systems führen. Des Weiteren sind zahlreiche permeationsfähige Wirkstoffe häufig in Gegenwart von Sauerstoff instabil bzw. die begrenzende Kleberschicht erlaubt den Zutritt von Sauerstoff, so dass die Systeme nur eine kurze Haltbarkeit aufweisen.

EP 0273 004 beschreibt ein transdermales oder topisches System, in welchem Aktivierungsmittel die Wirkstoffabgabe starten. In einer Ausführungsform kann die Barriere zwischen Haut und Wirkstoffschicht verändert werden. In der Ausführungsform stellt die permeable Membran ein Xerogel oder ein ionisches Gel dar, das für den Wirkstoff oder Bestandteile der Wirkstoffformulierung nur in hydratisierten Form permeabel ist. Das funktionstüchtige System enthält eine zusätzliche Kammer mit Wasser oder Pufferlösung die nach Aktivierung eine Hydrierung des Xerogels bzw. Erhöhung des Wassergehalts bewirken und somit die Poren der Membran für den Wirkstoff permeabel gestalten. Als Membranmaterial werden quervernetzte Polyacrylate genannt, als Aktivierungsmittel eine Base. Die beschriebene Ausführungsform ist in der Herstellung ausgesprochen aufwändig und der Funktionsmechanismus führt meist zu einer nicht sehr reproduzierbaren Abgabekinetik.

Der vorliegenden Erfindung liegt die Aufgabe zugrunde, verbesserte dermale oder transdermale therapeutische Systeme ohne die geschilderten Nachteile herzustellen.

In der Patentanmeldung GB 2 402 884 A wird ein transdermales Hautpflaster beschrieben, bei dem eine poröse Schicht auf die hautseitige Fläche des Wirkstoffreservoirs aufgebracht ist. Die poröse Schicht enthält Poren, deren Größe oder Verbindungen untereinander sich mit der Temperatur ändern. Die poröse Schicht kann aus Polymeren, Proteinen, gelbasierenden Materialien oder gewebten Materialien hergestellt sein. Die Poren der Schicht sind bei bestimmten Temperaturen permeabel für den Wirkstoff, bei niedrigeren Temperaturen jedoch impermeabel für den Wirkstoff. Bei normaler Hauttemperatur von ungefähr 33 °C sind die Poren für den Wirkstoff impermeabel. Erst unter Fieberbedingungen entsprechenden Temperaturen werden die Poren permeabel für den Wirkstoff.

Diese Aufgabe wird durch die vorliegende Erfindung gelöst, welche ein dermales oder transdermales System mit einem Reservoir, enthaltend mindestens einen Wirk-stoff, einer das Wirkstoffreservoir begrenzenden wirkstoffdurchlässigen Membran, und einer Verschlussschicht betrifft, wobei die Verschlussschicht für den mindestens einen Wirkstoff bei einer Temperatur unterhalb der Hauttemperatur impermeabel und bei einer Temperatur von Hauttemperatur oder darüber permeabel ist. Unter Hauttemperatur ist hierbei die Temperatur der menschlichen Haut zu verstehen, die abhängig von der Person und den Umweltbedingungen schwanken kann und im Bereich von 30 bis 35°C liegt. Insbesondere liegt die Hauttemperatur im Bereich von 31 bis 33°C, besonders bevorzugt bei etwa 32°C.

Durch die Erfindung ist es möglich, dermale und transdermale therapeutische Systeme mit höherer Lagerstabilität und somit auch Produktsicherheit herzustellen und in diesem Zuge die Auswahl der für die Systeme geeigneten Wirkstoffe erweitern zu können.

Da in dem erfindungsgemäßen System eine an der Hautseite der Membran vorgesehene zusätzliche Kleberschicht vermieden werden kann, ist es möglich, auch Wirkstoffmengen in höherer Dosis zu verabreichen.

Ein weiterer Vorteil des erfindungsgemäßen Systems liegt in der Herstellung von dermalen oder transdermalen Systemen, in denen sich der Wirkstoff während der Lagerung nicht in der Kleberschicht anreichern kann, womit eine zu hohe initiale Freisetzung des Wirkstoffs vermieden wird.

Das erfindungsgemäße dermale oder transdermale therapeutische System umfasst folgende Bestandteile gleicher oder unterschiedlicher Größe: Geschlossene und für die Bestandteile der Wirkstoffformulierung undurchlässige Deckschicht; flüssige oder feste Reservoirschicht enthaltend einen oder mehrere Wirkstoffe, wobei das wirkstoffhaltige Reservoir durch eine wirkstoffdurchlässige Membran, die hautseitig mit einer Verschlussschicht versehen ist, begrenzt wird. Im Prinzip ist es möglich, dass die Reservoirmatrix als Flüssigkeit, in Gelform oder als selbsttragender Feststoff vorliegt. Der Fachmann wird natürlich im Falle einer flüssigen oder fließenden Matrix geeignete Vorkehrungen treffen, um ein "Ausfließen" der Matrix bzw. des oder der enthaltenden Wirkstoffe zu verhindern. Beispiele sind etwa das Vorsehen einer festen, flüssigkeitsundurchlässigen Umhüllung, die auf der der Haut zugewandten Seite von einer wirkstoffdurchlässigen Membran begrenzt wird, oder das Eindicken der Matrix mit Hilfe geeigneter Gelbildner. Vorzugsweise handelt es sich um eine Matrix auf Polymerbasis.

Für die Herstellung der Reservoirmatrix eignen sich grundsätzlich alle Polymere, die bei der Herstellung von transdermalen Systemen eingesetzt werden und physiologisch unbedenklich sind. Die Polymere können ausgewählt sein aus der Gruppe umfassend Cellulose-Derivate, wie Ethylcellulose, Hydroxypropylcellulose oder Carboxymethylcellulose, Polyethylene, chlorierte Polyethylene, Polypropylene, Polyurethane, Polycarbonate, Polyacrylsäureester, Polyacrylate, Polymethacrylate, Polyvinylalkohole, Polyvinylchloride, Polyvinylidenchloride, Polyvinylpyrrolidone, Polyethylenterephthalate, Polytetrafluoroethylene, Ethylen/Propylen-Copolymere, Ethylen/Ethylacrylat-Copolymere, Ethylen/Vinylacetat-Copolymere, Ethylen/Vinylalkohol-Copolymere, Vinylchlorid/Vinylacetat-Copolymere, Vinylpyrrolidon/Ethylen/Vinylacetat-Copolymere, Kautschuke, gummiartige, synthetische Homo-, Co- oder Blockpolymere, Silicone, Silicon-Derivate und deren Mischungen bestehen. Beispielhaft können auch Polymere auf Basis von Styrol-Butadien-Styrol Block-co-polymeren, Polyisobutylen etc. verwendet werden.

Das wirkstoffhaltige Reservoir des Pflasters kann auch Hautdurchdringungsverstärker, Füllstoffe (wie Zinkoxid oder Kieselsäure), Lösungsvermittler, Vernetzer, Stabilisatoren, Emulgatoren, Konservierungsmittel, Antioxidationsmittel und/oder Lösemittel enthalten. Der Fachmann wird hierbei selbstverständlich berücksichtigen, dass die Stabilität der Verschlussschicht von den genannten Hilfsstoffen nicht beeinträchtigt wird.

Das System weist auf der Applikationsfläche (hautseitig) gegebenenfalls eine abziehbare Schutzschicht auf.

Die Verschlussschicht wird durch Einwirken des Benutzers von der impermeablen in die permeable Form überführt, was beim Anbringen des Systems auf der Haut geschieht. Dabei bewirkt die Temperaturerhöhung durch die Haut eine Erhöhung der Permeabilität der Verschlussschicht und somit eine Freisetzung des Wirkstoffes durch die wirkstoffdurchlässige Membran in die Hautschicht. Die Verschlussschicht kann im weiteren Verlauf die Funktion eines Permeationsverstärkers übernehmen und die Wirkstoffabgabe somit beschleunigen. Die bei einer Temperatur unterhalb der Hauttemperatur impermeable Versclussschicht ist vorzugsweise für Sauerstoff undurchlässig.

Des Weiteren kann die Verschlussschicht eine Eigenklebrigkeit besitzen, um das Systems ganzflächig auf der Haut zu fixieren.

Zur Verbesserung der Stabilität von Wirkstoffen, die dem oxidativen Abbau unterliegen, kann die Verschlussschicht mit einem oder mehreren Antioxidantien versehen sein.

Die Trägerschicht bzw. Deckschicht des Pflasters ist vorzugsweise für die in der wirkstoffhaltigen Schicht und in der Klebeschicht enthaltenen Stoffe, insbesondere für den Wirkstoff, undurchlässig und inert, und kann auf Polymeren, wie Polyester, z. B. Polyethylenterephthalat, Polyolefinen, wie Polyethylenen, Polypropylenen oder Polybutylenen, Polycarbonaten, Polyethylenoxiden, Polyurethanen, Polystyrolen, Polyamiden, Polyimiden, Polyvinylacetaten, Polyvinylchloriden, Polyvinylidenchloriden und/oder Copolymeren, wie Acrylnitril/Butadien/Styrol Copolymeren, gegebenenfalls enthaltend Papierfasern, Textilfasern und/oder deren Mischungen, basieren, die bei Bedarf metallisiert oder pigmentiert sein können. Die Trägerschicht bzw. Deckschicht des Pflasters kann auch aus einer Kombination aus Metallfolie und Polymerschicht bestehen. Die Dicke der Trägerschicht beträgt vorzugsweise 3 bis 100 µm.

In einer weiteren Ausführungsform kann das System mit einem Deckpflaster (Overtape) auf der Haut fixiert werden.

Weitere Merkmale der Erfindung ergeben sich aus der vorliegenden Beschreibung von Ausführungsbeispielen in Verbindung mit den Ansprüchen und der Figur. Die einzelnen Merkmale können bei einer Ausführungsform gemäß der Erfindung je für sich oder zu mehreren verwirklicht sein. Bei der nachfolgenden Erläuterung einiger Ausführungsbeispiele der Erfindung wird auf die beiliegende Figur Bezug genommen, die ein erfindungsgemäßes System in einer schematischen Darstellung zeigt.

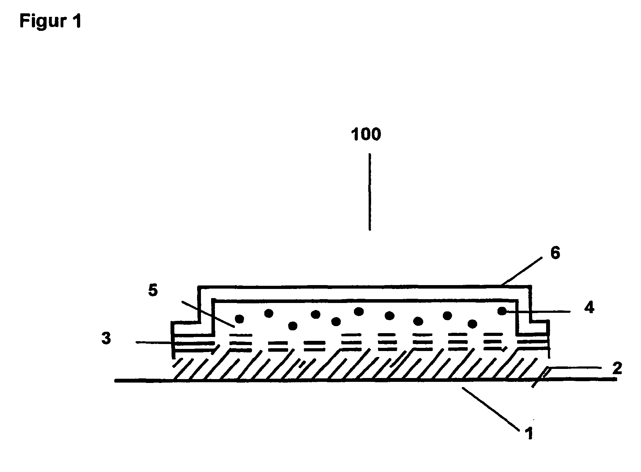

Figur 1 zeigt eine Ansicht einer bevorzugten erfindungsgemäßen Ausführungsform in Form eines flüssigkeitsgefüllten Reservoirsystems 100 mit begrenzender wirkstoffdurchlässiger Membran und Verschlussschicht. Die unterste Schicht 1 ist eine abziehbare Schutzschicht, welche vom Benutzer oder einer anderen Person vor dem Anbringen des Systems auf der Haut entfernt wird. Das applikationsfähige Pflaster wird als Ganzes auf der Haut des Patienten befestigt. Die hautseitige Verschlussschicht 2 ist mit der wirkstoffdurchlässigen Membran 3 verbunden und der Haut des Patienten zugewandt. Das den Wirkstoff 4 enthaltende Reservoir 5 wird auf der der Haut abwandten Seite von der undurchlässige Deckschicht 6 begrenzt. Die Deckschicht 6 ist mit der Membran 3 verbunden oder vorzugsweise verschweißt. Die der Deckschicht gegenüberliegende, freie Oberfläche wird im Folgenden als hautseitige Oberfläche des transdermalen Applikationssystems 100 bezeichnet.

Der beschriebene Aufbau des transdermalen Applikationssystems 100 dient dazu, einen oder mehrere der in dem Reservoir 5 enthaltenen Wirkstoffe 4 durch die wirkstoffdurchlässige Membran 3 und die daran anschließende hautseitige Verschlussschicht 2 hindurch in ausreichender Konzentration kontrolliert über einen längeren Zeitraum hinweg zu der Hautoberfläche zu transportieren, auf der das System befestigt ist.

Beispiele für geeignete Wirkstoffe sind Analgetika, µ-Opioid-Rezeptor-Antagonisten, anästhesierende Wirkstoffe, Parasympathomimetika, Parasympatholytika, Antiemetika, Emetika, Sympathomimetika, Hormone, Antimigränemittel, Antiallergika, Antikonvulsiva, Antidementiva, Antidepressiva, Betablocker, Alphablocker und Analeptika.

Bei den Analgetika kann es sich um Opioide handeln, wobei von diesen die reinen Agonisten, die gemischten Agonisten-Antagonisten, die Partialantagonisten und die reinen Antagonisten angegeben werden können. Reine Agonisten sind etwa Fentanyl, Remifentanil, Oxycodon und Methadon. Von den reinen Antagonisten können Naloxon und Naltrexon genannt werden. Ein gemischter Agonist-Antagonist ist z. B. Nalbuphin, ein Partialantagonist ist beispielsweise Buprenorphin. Salicylsäurederivate wie Acetylsalicylsäure, Etofenamat oder Diclofenac sind Beispiele für saure Analgetika.

Von den µ-Opioid-Rezeptor-Antagonisten können z. B. Almivopan und Methylnaltrexon genannt werden.

Von den anästhesierenden Wirkstoffen kommen etwa die Lokalanästhetika in Betracht, beispielsweise Lidocain, Tetracain oder Etidocain.

Beispiele für Parasympathomimetika sind die Cholinesterase-Hemmer, wobei von diesen insbesondere Physostigmin, Rivastigmin, Neostigmin, Donepezil und Galanthamin zu nennen sind.

Von den Parasympatholytika kann z. B. das Scopolamin angegeben werden.

Die Antiemetika können unter den Parasympatholytika, 5-HT3-Rezeptor-Antagonisten, wie Ondansetron und Granisetron, und Dopamin-D2-Rezeptor-Antagonisten, wie Domperidon, ausgewählt werden.

Von den Emetika kommen Dopamin-D2-Rezeptor-Agonisten, wie Apomorphin, in Betracht.

Von den Sympathomimetika kommen die Catecholamine in Betracht, wie Dobutamin. Ferner sind auch die β-2-Sympathomimetika zu nennen, wie Salbutamol, Fenoterol und Clenbuterol.

Die Hormone können z. B. unter Estradiol, Norelgestromin, Goserelin oder Buserelin ausgewählt sein.

Beispiele für Antimigräne-Mittel sind 5-HT1-Rezeptor-Agonisten, wie Triptane und von diesen insbesondere Zolmitriptan, Sumatriptan oder Naratriptan.

Als Antagonist für alpha-1-Adrenorezeptoren kann Tamsulosin genannt werden.

Ein Beispiel fürAntikonvulsiva ist das Gabapentin.

Als Antidementivum kann das Memantin genannt werden.

Von den Antihistaminika kommen die Antiallergika in Betracht, wie beispielsweise Mizolastin, Triprolidin und Desloratadin.

Von den Antidepressiva kann das Nortriptylin angegeben werden.

Ein geeigneter Betablocker ist etwa das Nebivolol.

Ein Beispiel für ein Analeptikum ist etwa das Methylphenidat.

Die Matrix kann auch mehr als einen Wirkstoff enthalten, etwa eine Kombination aus 2 Wirkstoffen, wie z. B. ein Parasympathomimetikum in Kombination mit einem Parasympatholytikum, insbesondere Physostigmin/Scopolamin, ein Analgetikum in Kombination mit einem Antiemetikum, wie Fentanyl in Kombination mit Granisetron, oder zwei Analgetika, wie einen µ-Rezeptor-Agonisten und einen µ-Rezeptor-Antagonisten, z. B. Fentanyl/Naloxon.

Besonders bevorzugt wird das erfindungsgemäße System für Wirkstoffe verwendet, die eine Temperaturinstabilität zeigen und für welche eine Lagertemperatur unterhalb von 26°C, insbesondere bei einer Temperatur im Bereich von 2 bis 8°C empfehlenswert ist.

Unter einer Verschlussschicht ist gemäß der vorliegenden Anmeldung eine Schicht zu verstehen, die bei einer Temperatur unterhalb der Hauttemperatur impermeabel und bei einer Temperatur von Hauttemperatur oder darüber permeabel ist. Unter Hauttemperatur ist die Temperatur der Haut zu verstehen, die abhängig von der Person und den Umweltbedingungen schwanken kann und im Bereich von 30 bis 35°C liegt. Insbesondere liegt die Hauttemperatur im Bereich von 31 bis 33°C, besonders bevorzugt im Bereich von 32°C. Bei dem erfindungsgemäßen System weist das Verschlussmaterial eine Schmelztemperatur im Bereich der Hauttemperatur auf, vorzugsweise im Bereich von 30 bis 35 °C und insbesondere von 31 bis 33 °C.

Es können auch Substanzgemische verwendet werden, wobei durch geeignete Wahl der Bestandteile und deren Mengenanteilen die gewünschte Schmelztemperatur eingestellt werden kann.

Die Substanz für die Verschlussschicht kann hierbei ausgewählt werden aus der Gruppe der Pflanzenfette, der tierischen Fette, der Fettsäuren, der Alkanole, der Mono-, Di- und Triglyceride von langkettigen gesättigten Fettsäuren (C₁₂H₂₄O₂ bis C₁₈H₃₆O₂) sowie mittelkettigen Triglyceriden (C₆H₁₂O₂ bis C₁₀H₂₀O₂), der Ester von langkettigen Alkoholen und Säuren, der natürlichen Harze, der hochmolekularen Paraffine, der Polyethylenglykolderivate von hydratisiertem Kastoröl, der Polyethylenglykolderivate des Tocopherols, der Polysaccaride und der Polymere der Acrylsäure. Insbesondere eignen sich homogene Mischungen aus genannten Substanzgruppen.

Aus der Gruppe der Pflanzenfette können genannt werden: Kakaobutter, Carnaubawachs, Cupuacubutter, Candelillawachs und Sheabutter. Homogene Mischungen der genannten Pflanzenfette können vorteilhaft sein.

Unter den Tierfetten sind zu nennen: Bienenwachs, Wollwachs. Walratersatz kann als synthetischer Ersatz für Walrat dienen.

Als Beispiel für eine Verbindung aus Mono-, Di- und Triglyceriden von langkettigen gesättigten Fettsäuren (C₁₀H₂₀O₂ bis C₁₈H₃₆O₂) kann Hartfett (Adeps solidus) genannt werden; aus der Gruppe der mittelkettigen Triglyceride (C₆H₁₂O₂ bis C₁₀H₂₀O₂) ist beispielhalft Neutralöl zu nennen, enthaltend insbesondere die Fettsäuren Caprylsäure und/oder Decansäure.

Aus der Gruppe der Alkanole können Dodecanol, Tridecylalkohol und Cetylalkohol genannt werden.

Als Beispiel für freie Fettsäuren dienen Undecensäure und Stearinsäure.

Als Ester von langkettigen Alkoholen und Säuren kann Palmitinsäuremyricylester genannt werden.

Kolophonium dient als Beispiel aus der Gruppe der natürlichen Harze.

Beispiele für Polysaccaride sind Xanthan, Guarkernmehl und Hyaluronsäure.

Ein Beispiel für Polyacrylsäure stellt Carbopol dar.

Tocophersolan stellt ein Beispiel für ein Material des Polyethylenglykolderivats von Tocopherol dar.

Bei dem erfindungsgemäßen System kann das Verschlussmaterial mit einer Schichtdicke von 50 bis 600 µm und insbesondere von 100 bis 500 µm vorgesehen sein. Besonders bevorzugt ist eine Schichtdicke von 200 bis 400 µm.

Dabei kann das erfindungsgemäße System durch ein Verschlussmaterial auf Basis von Pflanzenfett und Tierfett gekennzeichnet sein. Vorteilhaft ist die Zugabe eines Naturharzes zur Verbesserung der Klebrigkeit des Systems.

Erfindungsgemäß kann das Verschlussmaterial antioxidative Eigenschaften aufweisen, was vor allem bei Wirkstoffen, die in Gegenwart von Sauerstoff oxidieren, von Relevanz ist. Bevorzugt sind hierbei Pflanzenfette mit einem Anteil an Antioxidantien oder der Zusatz von Antioxidantien. Aus der Gruppe der Pflanzenfette ist als besonders bevorzugt Cupuacubutter, insbesondere Palmitinsäurymyricylester hervorzuheben; Tocophersolan als Antioxidans kann ebenfalls Verwendung finden.

Des Weiteren kann das erfindungsgemäße System durch ein Verschlussmaterial auf Basis von Hartfett (Adeps solidus) und Neutralöl gekennzeichnet sein. Die Mischung aus Hartfett und Neutralöl enthält vorzugsweise 60 bis 90 Gew.-% Hartfett und 10 bis 40 Gew.-% und noch bevorzugter 75 bis 90 Gew.-% Hartfett und 10 bis 25 Gew.% Neutralöl.

Bei dem erfindungsgemäßen System kann die Konsistenz des Verschlussmaterials durch Zugabe einer Fettsäure eingestellt werden. Vorteilhaft ist hierbei die Verwendung der Fettsäure Undecensäure oder Stearinsäure zu einem Anteil von 1 - 20 Gew.-%. Besonders bevorzugt ist ein Anteil von 5 - 10 Gew.-%. Des Weiteren können Alkanole wie Cetylalkohol, Tridecylalkohol oder Dodecanol zu einem Anteil von 1 - 20 Gew.-% hinzugefügt werden. Insbesondere ist ein Anteil von 5 - 10 Gew.-% vorteilhaft.

Des Weiteren kann das Verschlussmaterial des erfindungsgemäßen Systems aus einem Polymer unter Zusatz eines Hilfsstoffes bestehen. Besonders bevorzugt ist Polyacrylsäure unter Zusatz des Polyethylenglykolderivates von hydratisiertem Kastoröl.

Die wirkstoffdurchlässige Membran des erfindungsgemäßen Reservoirsystems besteht vorzugsweise aus einem inerten Polymer, das z. B. ausgewählt ist unter Polyethylenen, Polypropylenen, Polyvinylacetaten, Polyamiden, Ethylen/Vinylacetat-Copolymeren und/oder Siliconen. Die Dicke der Membran beträgt 5 bis 100 µm, vorzugsweise zwischen 10 bis 50 µm und besonders bevorzugt 15 bis 40 µm.

Die wirkstoffdurchlässige Membran weist vorzugsweise Poren auf, deren Größe im Bereich von 0,1 bis 50 µm liegen kann. Die Poren haben vorzugsweise eine Größe im Bereich von 0,2 bis 10 µm und besonders bevorzugt im Bereich von 0,5 bis 5 µm auf.

Dabei kann das erfindungsgemäße System mit einer Folie als Deckpflaster versehen sein, wobei die Folie das System allseitig überragt und zumindest in einer umlaufenden Zone mit einem Haftkleber versehen sein kann.

Die lösbare Schutzschicht des erfindungsgemäßen Reservoirsystems kannen aus Polyethylen, Polyester, Polyethylenterephthalat, Polypropylen, Polysiloxan, Polyvinylchlorid oder Polyurethan und gegebenenfalls aus behandelten Papierfasern, wie z. B. Zellophan, bestehen und gegebenenfalls vorzugsweise eine Silicon-, Fluorsilicon- oder Fluorkohlenstoffbeschichtung aufweisen.

Die erfindungsgemäßen dermalen und transdermalen therapeutischen Systeme können ganz allgemein so hergestellt werden, wie dies für die speziellen Ausführungsbeispiele in den folgenden Beispielen 1 und 2 beschrieben wird.

Nachstehend werden die Ausführungsbeispiele der Erfindung näher erläutert.

### Beispiel 1

Zur Herstellung der Verschlussschicht wird eine Mischung aus Hartfett (80 Gew.-%) und Neutralöl (20 Gew.-%) bei 75 °C geschmolzen und bei 40°C in flüssiger Phase gehalten. Palmitinsäuremyricylester wird in einer Menge von 2 Gew.-% homogen eingearbeitet. Die Beschichtung der wirkstoffdurchlässigen Membran (vorliegend mikroporöse Polyethylenmembran DSM Solupor) erfolgt auf einer kontinuierlichen Beschichtungsmaschine mittels eines Messergießers in einer Dicke von 250 µm.

Nach dem Beschichten wird die Membran bis zum Erhärten der Verschlussschicht bei 4 °C abgekühlt. Zur Herstellung des Reservoirsystems wird die beschichtete Membran mit einer herkömmlichen Siegelmaschine bei 175 °C und Druck unter Beibehaltung einer schmalen Befüllöffnung auf eine Deckschicht (Backing Folie) geschweißt. Tamsulosin Base (2 Gew.-%) wird in Ethanol (75 Gew.-%) gelöst und unter Zugabe von 23 Gew.-% Isopropylmyristat zu einer homogenen Lösung verrührt. Das Reservoir wird über die Befüllöffnung mit der wirkstoffhaltigen Lösung befüllt, verschweißt und ausgestanzt. Das System wird bei 2 bis 8°C gelagert. Nach Entfernen der Schutzfolie erfolgt die Fixierung des Systems auf der Haut mittels eines klebstoffbeschichteten Overtapes.

### Beispiel 2

Zur Herstellung der Verschlussschicht wird eine Mischung aus Bienenwachs (70 Gew.-%), Carnaubawachs (20 Gew.-%) und Kolophonium (10 Gew.-%) bei 110 °C geschmolzen und bei 45 °C in flüssiger Phase gehalten. Tocophersolan wird in einer Menge von 5 -10 Gew.-% zugegeben. Die flüssige Mischung wird bis zur Homogenität gerührt. Die Beschichtung der wirkstoffdurchlässigen Membran (vorliegend Polypropylen Celgard 2400) erfolgt auf einer kontinuierlichen Beschichtungsmaschine mittels eines Messergießers bei einer Dicke von 300 µm.

Nach dem Beschichten wird die Membran bis zum Erhärten der Verschlussschicht abgekühlt (4°C). Zur Herstellung des Reservoirsystems wird die beschichtete Membran mit einer herkömmlichen Siegelmaschine bei 175°C und Druck unter Beibehalt einer schmalen Befüllöffnung auf eine Deckschicht (Backing Folie) geschweißt. Physostigmin Base wird in einer ethanolischen Lösung gelöst. Hydroxypropylcellulose wird mit einem Anteil von 2,5 Gew.-% zugegeben; die Mischung lässt man bis zur vollständigen Quellung der Hydroxypropylcellulose stehen. Das Reservoir wird über die Befüllöffnung mit dem wirkstoffhaltigen Hydrogel befüllt, verschweißt und ausgestanzt. Das System wird bei einer Temperatur unter 25°C gelagert. Nach Entfernen der Schutzfolie erfolgt die Fixierung des Systems auf der Haut.

## Patentansprüche

1. Dermales oder transdermales therapeutisches System, das ein Reservoir (5) mit mindestens einem Wirkstoff (4), eine das Wirkstoffreservoir (5) begrenzende wirkstoffstoffdurchlässige Membran (3) und eine Verschlussschicht (2) aufweist, wobei das Material der Verschlussschicht (2) eine Schmelztemperatur im Bereich von 30 bis 35°C aufweist, und die Verschlussschicht (2) für den mindestens einen Wirkstoff (4) bei einer Temperatur unterhalb der Hauttemperatur impermeabel und bei einer Temperatur von Hauttemperatur oder darüber permeabel ist.

2. Dermales oder transdermales therapeutisches System nach Anspruch 1, worin die Schmelztemperatur aus dem Bereich von 31 bis 33 °C gewählt ist und insbesondere 32 °C beträgt.

3. Dermales oder transdermales therapeutisches System nach einem der Ansprüche 1 oder 2, wobei es sich bei dem Verschlussmaterial um Substanzen handelt ausgewählt aus der Gruppe der Pflanzenfette, der Tierfette, der Alkanole, der Fettsäuren, der Mono-, Di- und Triglyceride von langkettigen gesättigten Fettsäuren (C₁₂H₂₄O₂ bis C₁₈H₃₆O₂) sowie der mittelkettigen Triglyceride (C₆H₁₂O₂ bis C₁₀H₂₀O₂), der Ester von langkettigen Alkoholen und Säuren, der natürlichen Harze, der hochmolekularen Paraffine, Polyethylenglykolderivate von hydratisiertem Kastoröl, Polyethylenglykolderivate des Tocopherols, Polysaccharide und Polymere der Acrylsäure und deren Gemischen.

4. Dermales oder transdermales System nach einem der Ansprüche Anspruch 1 bis 3, wobei das Verschlussmaterial unter Kakaobutter und Cupuacubutter, Walratersatz, Bienenwachs, Hartfett, Neutralöl, Dodecanol, Cetylalkohol, Undecensäure, Palmitinsäuremyricylester, Kolophonium, Tocophersolan, Guarkernmehl und Carbopol und deren Gemischen ausgewählt ist.

5. Dermales oder transdermales therapeutisches System nach einem der vorhergehenden Ansprüche, wobei die Materialien für die Verschlussschicht (2) aus einer homogenen Mischung bestehen.

6. Dermales oder transdermales System nach einem der vorhergehenden Ansprüche, wobei die Verschlussschicht (2) unter Mischungen aus Pflanzen- und Tierfetten, vorzugsweise Pflanzen- und Tierfetten in Kombination mit Harzen ausgewählt sind.

7. Dermales oder transdermales System nach Anspruch 5 oder 6, wobei die Mischung aus Bienenwachs und Carnaubawachs besteht, wobei der Anteil an Bienenwachs im Bereich von 40 bis 95 Gew.-% und der Anteil an Carnaubawachs im Bereich von 5 bis 60 Gew.-% liegt.

8. Dermales oder transdermales therapeutisches System nach einem der vorhergehenden Ansprüche, wobei es sich bei den Substanzen für die Verschlussschicht (2) um eine homogene Mischung von Mono-, Di- und Triglyceriden von langkettigen gesättigten Fettsäuren (C₁₂H₂₄O₂ bis C₁₈H₃₆O₂) und mittelkettigen Triglyceriden (C₆H₁₂O₂ bis C₁₀H₂₀O₂) handelt.

9. Dermales oder transdermales therapeutisches System nach einem der vorhergehenden Ansprüche, wobei die Mischung aus Hartfett und Neutralöl besteht, in der das Hartfett in einem Anteil von 60 bis 90 Gew.-% und das Neutralöl in einem Anteil von 10 bis 40 Gew.-% enthalten ist.

10. Dermales oder transdermales therapeutisches System nach einem der vorhergehenden Ansprüche, wobei die Verschlussschicht (2) 0,1 bis 10 Gew.-% Antioxidantien enthält.

11. Dermales oder transdermales therapeutisches System nach Anspruch 10, wobei es sich bei dem Antioxidans oder den Antioxidantien um Polyethylenglykolderivate des Tocopherols, insbesondere Tocophersolan, handelt.

12. Dermales oder transdermales therapeutisches System nach einem der vorhergehenden Ansprüche, wobei die Verschlussschicht (2) bei einer Temperatur von Hauttemperatur oder darüber permeationsfördernde Eigenschaften aufweist.

13. Dermales oder transdermales therapeutisches System nach einem der vorhergehenden Ansprüche, wobei die Verschlussschicht (2) haftklebende Eigenschaften aufweist.

14. Dermales oder transdermales therapeutisches System nach einem der vorhergehenden Ansprüche, wobei die Schichtdicke der Verschlussschicht (2) im Bereich von 50 bis 600 µm, insbesondere 100 bis 500 µm und bevorzugt von 200 bis 400 µm liegt.

15. Dermales oder transdermales therapeutisches System nach einem der vorhergehenden Ansprüche, wobei die den mindestens einen Wirkstoff (4) enthaltende Schicht als festes, halbfestes oder flüssiges Reservoir (5) ausgebildet ist.

16. Dermales oder transdermales therapeutisches System nach einem der Ansprüche 1 bis 15 zur Abgabe mindestens eines Wirkstoffs (4) an die Haut.

## Claims

1. Dermal or transdermal therapeutic system comprising a reservoir that contains at least one active substance, an active substance-permeable membrane which delimits the active substance reservoir, and a closing layer, the material of the closing layer having a melting temperature in the range from 30°C to 35°C, and the closing layer being impermeable to the at least one active substance at a temperature lying below the skin temperature while being permeable at skin temperature or above.

2. Dermal or transdermal therapeutic system according to claim 1,
wherein the melting temperature is selected from the range from 31°C to 33 °C and is in particular 32 °C.

3. Dermal or transdermal therapeutic system according to claim 1 or 2, wherein the closing material is selected from substances from the group of vegetable fats, animal fats, alkanols, fatty acids, mono-, di- and triglycerides of long-chain saturated fatty acids (C₁₂H₂₄O₂ to C₁₈H₃₆O₂) as well as medium-chain triglycerides (C₆H₁₂O₂ to C₁₀H₂₀O₂), esters of long-chain alcohols and acids, natural resins, high-molecular paraffines, polyethylene glycol derivatives of hydrated castor oil, polyethylene glycol derivatives of tocopherol, polysaccharides and polymers of acrylic acid and mixtures thereof.

4. Dermal or transdermal system according to one of claims 1 to 3,
wherein the closing material is selected among cocoa butter and cupuacu butter, spermaceti substitutes, beeswax , hard fat, neutral oil, dodecanol, cetyl alcohol, undecenoic acid, palmitic acid myricyl ester, colophony, tocophersolan, guar flour and Carbopol and the mixtures thereof.

5. Dermal or transdermal therapeutic system according to one of the preceding claims, wherein the materials for the closing layer consist of a homogeneous mixture.

6. Dermal or transdermal system according to one of the preceding claims, wherein the closing layer is selected among mixtures of vegetable fats and animal fats, preferably vegetable fats and animal fats in combination with resins.

7. Dermal or transdermal system according to claim 5 or 6, wherein the mixture consists of bees wax and carnauba wax, with the proportion of bees wax being in the range of 40 to 95 % by weight and the proportion of carnauba wax being in the range of 5 to 60 % by weight.

8. Dermal or transdermal therapeutic system according to one of the preceding claims, wherein the substances for the closing layer are a homogeneous mixture of mono-, di- and triglycerides of long-chain saturated fatty acids (C₁₂H₂₄O₂ to C₁₈H₃₆O₂) and medium-chain triglycerides (C₆H₁₂O₂ to C₁₀H₂₀O₂).

9. Dermal or transdermal therapeutic system according to one of the preceding claims, wherein the mixture consists of hard fat and neutral oil, with the hard fat being contained in a proportion of 60 to 90 % by weight and the neutral oil being contained in a proportion of 10 to 40 % by weight.

10. Dermal or transdermal therapeutic system according to one of the preceding claims, wherein the closing layer comprises 0.1 to 10 % by weight of antioxidants.

11. Dermal or transdermal therapeutic system according to one of the preceding claims, wherein the antioxidant or the antioxidants are polyethylene glycol derivatives of tocopherol, in particular tocophersolan.

12. Dermal or transdermal therapeutic system according to one of the preceding claims, wherein the closing layer has permeation-enhancing properties at skin temperature or above.

13. Dermal or transdermal therapeutic system according to one of the preceding claims, wherein the closing layer has pressure-sensitive adhesive properties.

14. Dermal or transdermal therapeutic system according to one of the preceding claims, wherein the layer thickness of the closing layer lies in the range of 50 µm to 600 µm, in particular of 100 µm to 500 µm, and preferably of 200 µm to 400 µm.

15. Dermal or transdermal therapeutic system according to one of the preceding claims, wherein the layer comprising the at least one active substance is formed as a solid, semi-solid or liquid reservoir.

16. Dermal or transdermal therapeutic system according to one of the claims 1 to 15 for the release of at least one active substance to the skin.

## Revendications

1. Système thérapeutique dermique ou transdermique, qui comprend un réservoir (5) comprenant au moins une substance active (4), une membrane (3) perméable à la substance active et délimitant le réservoir (5) de substance active et une couche d'obturation (2), dans lequel la matière de la couche d'obturation (2) présente une température de fusion se situe dans la plage allant de 30 à 35°C, et la couche d'obturation (2) est imperméable pour la au moins une substance active (4) à une température inférieure à la température de la peau et perméable à une température égale ou supérieure à la température de la peau.

2. Système thérapeutique dermique ou transdermique selon la revendication 1, dans lequel la température de fusion est choisie dans la plage allant de 31 à 33°C et atteint en particulier 32°C.

3. Système thérapeutique dermique ou transdermique selon l'une des revendications 1 ou 2, dans lequel la matière d'obturation est composée de substances choisies dans le groupe constitué par les graisses végétales, les graisses animales, les alcanols, les acides gras, les mono-, di- et triglycérides d'acides gras saturés à longues chaînes (C₁₂H₂₄O₂ à C₁₈H₃₆O₂) ainsi que les triglycérides à chaînes moyennes (C₆H₁₂O₂ à C₁₀H₂₀O₂), les esters d'alcools et d'acides à longues chaînes, les résines naturelles, les paraffines de poids moléculaire élevé, les dérivés de polyéthylène glycol de l'huile de ricin hydrogénée, les dérivés de polyéthylène glycol du tocophérol, les polysaccharides et les polymères d'acide acrylique et leurs mélanges.

4. Système thérapeutique dermique ou transdermique selon l'une des revendications 1 à 3, dans lequel la matière d'obturation est choisie parmi le beurre de cacao, le beurre de cupuaçu, l'ersatz de blanc de baleine, la cire d'abeilles, la graisse dure, l'huile neutre, le dodécanol, l'alcool cétylique, l'acide undécylénique, l'ester myricylique d'acide palmitique, la colophane, la tocophersolane, la farine de graine de guar et Carbopol et leurs mélanges.

5. Système thérapeutique dermique ou transdermique selon l'une des revendications précédentes, dans lequel les matières pour la couche d'obturation (2) sont composées d'un mélange homogène.

6. Système dermique ou transdermique selon l'une des revendications précédentes, dans lequel la couche d'obturation (2) est choisie parmi les mélanges constitués de graisses végétales et animales, de préférence de graisses végétales et animales en combinaison avec des résines.

7. Système dermique ou transdermique selon la revendication 5 ou 6, dans lequel le mélange se compose de cires d'abeilles et de cire de carnauba, où la proportion de cire d'abeilles se situe dans la plage allant de 40 à 95 % en poids et la proportion de cire de carnauba se situe dans la plage allant de 5 à 60 % en poids.

8. Système thérapeutique dermique ou transdermique selon l'une des revendications précédentes, dans lequel les substances destinées à la couche d'obturation (2) sont un mélange homogène de mono-, di- et triglycérides d'acides gras saturés à longues chaînes (C₁₂H₂₄O₂ à C₁₈H₃₆O₂) et de triglycérides à chaînes moyennes (C₆H₁₂O₂ à C₁₀H₂₀O₂).

9. Système thérapeutique dermique ou transdermique selon l'une des revendications précédentes, dans lequel le mélange se compose de graisse dure et d'huile neutre, où la graisse dure est présente en une proportion de 60 à 90 % en poids et l'huile neutre en une proportion de 10 à 40 % en poids.

10. Système thérapeutique dermique ou transdermique selon l'une des revendications précédentes, dans lequel la couche d'obturation (2) comprend 0,1 à 10 % en poids d'antioxydants.

11. Système thérapeutique dermique ou transdermique selon la revendication 10, dans lequel l'antioxydant ou les antioxydants est ou sont des dérivés de polyéthylène glycol du tocophérol, en particulier de la tocophersolane.

12. Système thérapeutique dermique ou transdermique selon l'une des revendications précédentes, dans lequel la couche d'obturation (2) présente des propriétés favorisant la perméation à une température égale ou supérieure à la température de la peau.

13. Système thérapeutique dermique ou transdermique selon l'une des revendications précédentes, dans lequel la couche d'obturation (2) présente des propriétés adhésives.

14. Système thérapeutique dermique ou transdermique selon l'une des revendications précédentes, dans lequel l'épaisseur de la couche d'obturation (2) se situe dans la plage allant de 50 à 600 µm, en particulier de 100 à 500 µm et de préférence de 200 à 400 µm.

15. Système thérapeutique dermique ou transdermique selon l'une des revendications précédentes, dans lequel la couche comprenant la au moins une substance active (4) prend la forme d'un réservoir (5) solide, semi-solide ou liquide.

16. Système thérapeutique dermique ou transdermique selon l'une des revendications 1 à 15, destiné à la libération d'au moins une substance active (4) sur la peau.
